(19) Europäisches Patentamt
European Patent Office
Office européen des brevets

(11) Veröffentlichungsnummer: **0 279 219 B1**

# (12) EUROPÄISCHE PATENTSCHRIFT

(45) Veröffentlichungstag der Patentschrift: **03.10.90**

(51) Int. Cl.⁵: **A01N 43/64**

(21) Anmeldenummer: **88101005.2**

(22) Anmeldetag: **25.01.88**

(54) **Mittel gegen Protozoen bei Insekten.**

(30) Priorität: **03.02.87 DE 3703105**

(43) Veröffentlichungstag der Anmeldung: **24.08.88 Patentblatt 88/34**

(45) Bekanntmachung des Hinweises auf die Patenterteilung: **03.10.90 Patentblatt 90/40**

(84) Benannte Vertragsstaaten: **BE CH DE ES FR GB IT LI NL**

(56) Entgegenhaltungen:
**DE-A- 2 313 721**
**DE-A- 2 650 014**
**DE-B- 2 413 722**

(73) Patentinhaber: **BAYER AG, D-5090 Leverkusen 1 Bayerwerk(DE)**

(72) Erfinder: **Mehlhorn, Heinz, Prof. Dr., Steinstrasse 12, D-4040 Neuss-Üdesheim(DE)**
Erfinder: **Schmahl, Günter, Dr., Markstrasse 329, D-4639 Bochum(DE)**

EP 0 279 219 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft die Verwendung von bekannten Triazintrionen zur Herstellung von Mitteln gegen Protozoen bei Insekten.

Zu den Protozoen gehören Parasiten, die bei Insekten (z.B. Nosema apis bei Bienen) weit verbreitet sind. Es sind davon vom Menschen gehaltene Nutzinsekten (z.B. Bienen oder Seidenraupen), aber auch im Labor gezüchtete Insekten sowie alle freilebenden Insekten betroffen. Die Parasiten schädigen die Wirtstiere durch Zerstörung ihrer Organe. Zusammen mit anderen Parasiten (z.B. Varroamilben bei Bienen), können sie erhebliche Schädigungen der Wirtstiere hervorrufen die bis zu deren Tod gehen können. Bei der Honigbiene rufen sie erheblichen Schaden durch Verminderung der Honigproduktion, Schwächung und Absterben der Völker hervor. Bei Laborzuchten von Insekten, die zur Sammlung genetischen Materials und Erhaltung von Arten gehalten werden, sind oft nur wenige Tiere vorhanden mit deren Absterben wertvolle Informationen verloren gehen.

Mittel gegen Protozoen bei Insekten gibt es bis jetzt noch nicht.

Es wurde gefunden, daß die bekannten Triazintrione der Formel (I)

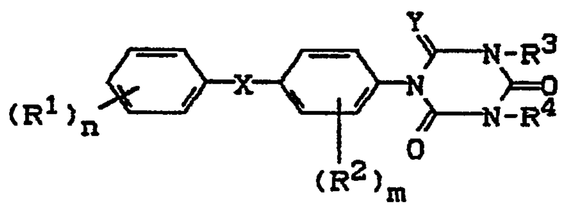

(I)

in welcher

X für O oder S steht,

Y für O oder S steht,

$R^1$ für gleiche oder verschiedene Reste aus der Gruppe Halogen, Nitro, CN, Amino, Alkyl, Halogenalkyl, Alkoxy, Halogenalkoxy, Alkylthio, Halogenalkylthio, Alkylsulfonyl, Alkylsulfinyl, Halogenalkylsulfonyl, Halogenalkylsulfinyl, Acyl, Carboxy, Carbonylamino, Carbonylalkoxy, Carbamoyl, Sulfamoyl steht,

$R^2$ für gleiche oder verschiedene Reste aus der bei $R^1$ angegebenen Gruppe von Substituenten,

n und m für ganze Zahlen von 0 bis 3 stehen,

$R^3$ für Wasserstoff oder Alkyl steht,

$R^4$ für Wasserstoff oder Alkyl steht,

zur Bekämpfung von Protozoen bei Insekten verwendet werden können.

Die Verbindungen der Formel (I) sind bekannt oder können nach an sich bekannten Methoden hergestellt werden (DE-OS 2 413 722; DE-OS 2 718 799, US-P 4 219 552).

Bevorzugt sind Verbindungen der Formel (I) in welcher

X und Y für O stehen,

$R^1$ und $R^2$ unabhängig voneinander für gleiche oder verschiedene Reste aus der Gruppe Halogen, insbesondere Fluor, Chlor, Brom, Nitro, CN, Amino, $C_{1-4}$-Alkyl, insbesondere Methyl oder Ethyl, $C_{1-4}$-Halogenalkyl, insbesondere Trifluormethyl, Trichlormethyl, Fluor-chlorethyl, $C_{1-4}$-Alkoxy, insbesondere Methylendioxy, Isopropoxy, Methoxy, $C_{1-4}$-Halogenalkoxy, insbesondere Trifluormethoxy, Difluormethylendioxy, Tetrafluorethylendioxy, $C_{1-4}$-Alkylthio, insbesondere Methylmercapto, Ethylmercapto, $C_{1-4}$-Halogenalkylthio, insbesondere Trifluormethylthio, $C_{1-4}$-Alkylsulfonyl, insbesondere Methylsulfonyl, $C_{1-4}$-Alkylsulfinyl, $C_{1-4}$-Halogenalkylsulfonyl une -sulfinyl, insbesondere Trifluormethylsulfonyl, $C_{1-4}$-Acyl, insbesondere Acetyl, Propionyl, Benzoyl, $C_{1-4}$-Carbonylalkoxy, insbesondere Methoxycarbonyl, Ethoxycarbonyl, stehen,

n und m für ganze Zahlen von 0 bis 2 stehen,

$R^3$ und $R^4$ unabhängig voneinander für Wasserstoff oder $C_{1-4}$-Alkyl, insbesondere Methyl stehen.

Besonders bevorzugt werden Verbindungen der Formel (I) eingesetzt, in welcher

$R^4$ für Wasserstoff steht,

$R^3$ für $C_{1-4}$-Alkyl steht,

Y für Sauerstoff oder Schwefel steht,

X für Sauerstoff steht,

$R^2$ für gleiche oder verschiedene Reste aus der Gruppe $C_{1-4}$-Alkyl, Halogen wie insbesondere Chlor oder Brom, $C_{1-4}$-Alkoxy und $C_{1-2}$-Halogenalkyl steht,

$R^1$ für gleiche oder verschiedene Reste aus der Gruppe Halogen insbesondere Chlor, Brom, $NO_2$, $C_{1-4}$-Alkyl, $C_{1-2}$-Halogenalkyl, $C_{1-4}$-Alkylmercapto, das gegebenenfalls durch Halogen substituiert ist, $C_{1-4}$-Alkoxy, das gegebenenfalls durch Halogen substituiert ist, $C_{1-4}$-Alkylsulfinyl, das gegebenenfalls durch Halogen substituiert ist, $C_{1-4}$-Alkylsulfonyl, das gegebenenfalls durch Halogen substituiert ist, steht,

n und m für ganze Zahlen von 0 bis 2 stehen.

Besonders bevorzugt sind Verbindungen der Formel (I), in welcher
$R^4$ für Wasserstoff steht,
$R^3$ für Methyl steht,
X und Y für Sauerstoff stehen,
$R^2$ für gleiche oder verschiedene Reste aus der Gruppe Chlor, Brom, Methyl, Trifluormethyl, Methoxy, Ethoxy steht,
$R^1$ für insbesondere in 4-Stellung gleiche oder verschiedene Reste aus der Gruppe Trifluormethylmercapto, Trifluormethylsulfinyl, Trifluormethylsulfonyl, Trifluormethoxy steht,
n und m unabhängig voneinander für 0 oder 1 stehen.

Folgende Verbindungen seien beispielhaft genannt, ohne die Erfindung in irgendeiner Weise einzuschränken:
1-[3,5-Dichlor-4-(4'-trifluormethoxy-phenoxy)-phenyl]-3-methyl-1,3,5-triazin-2,4,6(1H, 3H, 5H)-trion,
1-[3,5-Dichlor-4-(4'-trifluormethylthio-phenoxy)-phenyl]-3-methyl-1,3,5-triazin-2,4,6(1H, 3H, 5H)-trion,
1-[4-(4'-Trifluormethylthio-phenoxy)-phenyl]-3-methyl-1,3,5-triazin-2,4,6(1H, 3H, 5H)-trion,
1-[3-Chlor-5-brom-4-(4'-trifluormethylthio-phenoxy)-phenyl]-3-methyl-1,3,5-triazin-2,4,6(1H, 3H, 5H)-trion,
1-[3,5-Dibrom-4-(4'-trifluormethylthio-phenoxy)-phenyl]-3-methyl-1,3,5-triazin-2,4,6(1H, 3H, 5H)-trion,
1-[3,5-Dichlor-4-(3'-methyl-4'-trifluormethylthiophenoxy)-phenyl]-3-methyl-1,3,5-triazin-2,4,6(1H, 3H, 5H)-trion,
1-[4-(4'-Trifluormethylsulfonyl-phenoxy)-phenyl]-3-methyl-1,3,5-triazin-2,4,6(1H, 3H, 5H)-trion,
1-[3,5-Dichlor-4-(4'-trifluormethylsulfonyl-phenoxy)-phenyl]-3-methyl-1,3,5-triazin-2,4,6(1H, 3H, 5H)-trion,
1-[3-Chlorp-5-brom-4-(4'-trifluormethylsulfonyl-phenoxy)-phenyl]-3-methyl-1,3,5-triazin-2,4,6(1H, 3H, 5H)-trion,
1-[3,5-Dichlor-4-(2'-Chlor-4'-trifluormethylsulfonylphenoxy)-phenyl]-3-methyl-1,3,5-triazin-2,4,6(1H, 3H, 5H)-trion,
1-[3-Methoxy-4-(4'-trifluormethylthio-phenoxy)-phenyl]-3-methyl-1,3,5-triazin-2,4,6-(1H, 3H, 5H)-trion,
1-[4-(4'-Trifluormethylsulfinyl-phenoxy(-phenyl]-3-methyl-1,3,5-triazin-2,4,6(1H, 3H, 5H)-trion,
1-[3,5-Dichlor-4-(4'-trifluormethylsulfinyl-phenoxy)-phenyl]-3-methyl-1,3,5-triazin-2,4,6(1H, 3H, 5H)-trion,
1-[4-(3'-Trifluormethylsulfonyl-phenoxy)-3,5-dimethylphenyl]-3-methyl-1,3,5-triazin-2,4,6(1H,, 3H, 5H)-trion,
1-[3-Chlor-4-(4'-trifluormethylsulfonyl-phenoxy)-phenyl]-3-methyl-1,3,5-triazin-2,4,6(1H, 3H, 5H)-trion,
1-[3-Methyl-4-(4'-trifluormethylthio-phenoxy)-phenyl]-3-methyl-1,3,5-triazin-2,4,6-(1H, 3H, 5H)-trion,
1-[3,5-Dichlor-4-(2'-methyl-4'-trifluormethylsulfonylphenoxy)phenyl]-3-methyl-1,3,5-triazin-2,4,6(1H, 3H, 5H)-trion,
1-[3-Chlor-5-methyl-4-(2'-chlor-4'-trifluormethylsulfonyl-phenoxy)-phenyl]-3-methyl-1,3,5-triazin-2,4,6(1H, 3H, 5H)-trion,
1-[4-(4'-Trifluormethylsulfonyl-phenoxy)-3,5-dimethylphenyl]-3-methyl-1,3,5-triazin-2,4,6(1H, 3H, 5H)-trion,
1-[3-Chlor-5-methyl-4-(4'-trifluormethylsulfonylphenoxy)-phenyl]-3-methyl-1,3,5-triazin-2,4,6(1H, 3H, 5H)-trion,
1-[3-Chlor-4-(2'-Chlor-4'-trifluormethylthio-phenoxy)-phenyl]-3-methyl-1,3,5-triazin-2,4,6(1H, 3H, 5H)-trion,
1-[3-Brom-4-(4'-trifluormethylsulfonyl-phenoxy)-phenyl]-3-methyl-1,3,5-triazin-2,4,6(1H, 3H, 5H)-trion,
1-[3-Chlor-5-trifluormethyl-4-(4'-trifluormethylsulfonyl-phenoxy)-phenyl]-3-methyl-1,3,5-triazin-2,4,6(1H, 3H, 5H)-trion,
1-[3,5-Dichlor-4(4'-trifluormethylsulfonyl-phenoxy)-phenyl]-3-methyl-2-thioxo-4,6-dioxo-1,3,5(1H, 3H, 5H)-triazin,
1-[3-Methyl-4-(4'-trifluormethylthio-phenoxy)-phenyl]-3-methyl-2-thioxo-4,6-dioxo-1,3,5-(1H, 3H, 5H)-triazin.
1-[3-Methyl-4-(6-trifluormethyl-benzthiazol-2-yloxy)-phenyl]-3-methyl-1,3,5-triazin-2,4,6-(1H, 3H, 5H)-trion.

Ganz besonders hervorgehoben sei Toltrazuril common name für 1-[3-Methyl-4-(4'-trifluormethylthio-phenoxy)-phenyl]-3-methyl-1,3,5-triazin-2,4,6(1H, 3H, 5H)-trion.

Es was bekannt, daß sich die Triazintrione der Formel (I) zur Bekämpfung von Coccidien der Säugetiere und des Geflügels einsetzen lassen. Es war nichts darüber bekannt, daß sie auch eingesetzt werden können, um Protozoen bei Insekten zu bekämpfen. Dies konnte auch nicht erwartet werden da diese Verwendung auch für andere Coccidiosemitteln nicht bekannt ist.

Zu den Protozoen die als Parasiten bei Insekten auftreten gehören die Schädlinge des Stamms Microsporida, insbesondere der Gattung Nosema. Besonders genannt sei Nosema apis.

Zu den Insekten gehören die vom Menschen gehaltenen Nutz- und Zuchtinsekten wie z.B. Honigbienen, Seidenraupen, Schlupfwespen aber auch alle in Laborzuchten gehaltenen Insekten die entweder zu Versuchszwecken oder zur Sammlung genetischen Materials gehalten werden.

Die Verbindungen der Formel (I) lassen sich bei allen Entwicklungsstadien der Insekten anwenden.

Die Behandlung kann auch mit Mitteln durchgeführt werden, die neben den angegebenen Wirkstoffen Wirkstoffe gegen andere Schädlinge enthalten. So ist z.B. bei Bienen eine kombinierte Behandlung gegen Nosema und Varroa jacobsoni möglich wenn die Mittel neben den angegebenen Wirkstoffen z.B. synthetische Phosphorsäureester wie Coumaphos, Malathion, Formamidine wie Chlordimeform, Phenothiazine wie Promazin, synthetische Pyrethroide wie Flumethrin, Cyfluthrin, Cyhalothrin oder Amitraz oder Cymiazol enthalten.

Die Behandlung der Protozoen auf den Insekten kann auf verschiedene Weise erfolgen:

1. durch direkten Kontakt mit dem Wirkstoff. Dazu wird dieser z.B. versprüht, verstäubt, verräuchert, verdampft, verdunstet oder in Trägerstoffe, die mit den Insekten in Kontakt kommen, eingearbeitet oder auf die Trägerstoffe aufgebracht,
2. durch eine systemische Wirkung über die Hämolymphe der Insekten. Dazu wird der Wirkstoff z.B. mit dem Futter oder Trinkwasser angeboten, oder bei volkbildenden Insekten in den Stock gegossen oder gespritzt.

Die Behandlung kann prinzipiell ganzjährig erfolgen.

Da Protozoen in der warmen Jahreszeit verstärkt auftreten, ist eine Behandlung zu Beginn er warmen Jahreszeit besonders bevorzugt.

Wird der Wirkstoff verdampft, verdunstet oder ist er in Trägerstoffe eingearbeitet, wird bevorzugt ganzjährig behandelt.

Bei Honigbienen wird die Behandlung besonders vorteilhaft zum Zeitpunkt der Wintereinfütterung oder in der brutfreien Zeit durchgeführt.

Weiter kann bei Honigbienen das Bienenvolk als Kunstschwarm behandelt werden. Dies kann auch während der Brutperiode erfolgen.

Mittel, die versprüht werden, enthalten den Wirkstoff in Konzentrationen von 0,1 - 50 Gew.-%, bevorzugt von 0,3 - 20 Gew.-%.

Diese Mittel werden entweder direkt oder nach weiterem Verdünnen, bevorzugt, mit Wasser, angewendet. Bei direkter Anwendung der Mittel ist die Ausbringung im ultra-low-volume-(ULV-)Verfahren mit dafür geeigneten üblichen Geräten bevorzugt. Die Mittel können auch unter Zuhilfenahme elektrostatischer Aufladung versprüht werden.

Die Mittel können vor der Anwendung auf Wirkstoff-Konzentrationen von $10^{-4}$ - 2 Gew.-%, bevorzugt $10^{-3}$ - 0,5 Gew.-%, verdünnt werden. Sie werden konventionell mit üblichen Geräten wie Rückenspritze, Kolbenpumpe, Lackierpistole versprüht.

Mit diesen Mitteln werden entweder die Insekten oder ihre Wohnung oder Teile davon oder ihre Umgebung behandelt.

Die Mittel enthalten den Wirkstoff neben Verdünnungsmitteln und/oder Emulgatoren, die in den angewendeten Konzentrationen insektenverträglich sind.

Geeignete Verdünnungsmittel sind Wasser, Alkohole, wie Methanol, Ethylalkohol, Propanol, Isopropylalkohol, n-Butylalkohol, Amylalkohol, Octanol;
Glykole, wie Propylenglykol, 1,3-Butylenglykol, Ethylenglykol, Dipropylenglykolmonomethylether;
Diethylenglycolmonomethylether;
Glycerin;
aromatische Alkohole wie Benzylalkohol;
Carbonsäureester, wie z. B. Ethylacetat, Benzylbenzoat, Butylacetat, Propylencarbonat, Milchsäureethylester;
aliphatische Kohlenwasserstoffe, Öle, wie z.B. Baumwollsaatöl, Erdnußöl, Maiskeimöl, Olivenöl, Ricinusöl, Sesamöl;
Ketone, wie z. B. Aceton und Methylethylketon;
synth. Mono- und Triglyceride mit natürlichen Fettsäuren.

Weiterhin sind u. a. Verbindungen wie Dimethylsulfoxid, Dimethylacetamid, Dimethylformamid, N-Methylpyrrolidon, Dioxan, 2-Dimethyl-4-oxymethyl-1,3-dioxolan gut als Verdünnungsmittel geeignet.

Besonders geeignet sind Wasser sowie niedere Alkohole mit bis zu 8 Kohlenstoffatomen im Molekül, sowie niedere Ketone wie Methylethylketon und Ether des Ethylenglykols und des Propylenglykols.

Es können bei der Herstellung der erfindungsgemäß verwendbaren Mittel ein oder mehrere Verdünnungsmittel eingesetzt werden.

Geeignete Emulgatoren sind:
anionaktive Tenside, wie Na-Laurylsulfat, Fettalkoholethersulfate, Mono/Dialkylpolyglykoletherorthophosphorsäureester-Monoethanolaminsalze, Calciumalkylarylsulfonat,
kationaktive Tenside, wie Cetyltrimethylammoniumchlorid,
ampholytische Tenside, wie Di-Na-N-lauryl-β-iminodipropionat oder Lecithin,
nicht ionogene Tenside, z. B. polyoxyethyliertes Ricinusöl, polyoxyethyliertes Sorbitan-Monooleat oder

Sorbitan-Monostearat, Glycerinmonostearat, Polyoxyethylenstearat, Alkylphenolpolyglykolether.

Bevorzugt seien als Emulgatoren genannt:
nicht-ionische, wasserlösliche Emulgatoren mit einem HLB (hydrophilic/lipophilic-balance-Wert) größer als 10, z.B. Emulgator NP 10® (Bayer AG), Alkylarylpolyglykolether; Renex 678® (Atlas Chemical Industries), Polyoxyethylenalkylarylether; Tween 40® (Atlas), Polyoxyethylensorbitanmonopalmitat; Myri 53® (Atlas), Polyoxyethylenstearat; Atlas G 3707®, Polyoxyethylenlaurylether; Atlas G 3920®, Polyoxyethylenoleylether; Atlas G 9046 T®, Polyoxyethylenmannitanmonolaurat; Emulgator 1371 B® (Bayer AG), Alkylpolyglykolether; Emulgator 1736® (Bayer AG), Alkylpolyglykolether (Oleylpolyglykolether); Emulgator OX® (Bayer AG), Alkylpolyglykolether (Dodecylpolyglykolether); Ninox BM-2® (Stepan Chemical Co.), ethoxyliertes Nonylphenol; Triton X-100® (Rohm und Haas Co.), Isooctylphenolpolyethoxyethanol; Cremophor ET®, polyoxyethyliertes Ricinusöl.

Die Emulgatoren sind in den erfindungsgemäßen Mitteln in Konzentrationen vom bis zu 10-fachen, bevorzugt bis zu 5-fachen, des eingesetzten Wirkstoffe enthalten. Die Verdünnungsmittel werden jeweils zur gewünschten Endkonzentration ergänzt.

Die Mittel werden hergestellt durch Lösen des oder der Wirkstoffe im Lösungsmittel oder in einem Emulgator oder Emulgator-Lösungsmittelgemisch, falls notwendig, unter Erwärmung. Erforderlichenfalls erfolgt eine weitere Verdünnung der Mittel mit Wasser auf die gewünschte Konzentration.

Die Mittel zum Stäuben enthalten den Wirkstoff neben herkömmlichen insektenverträglichen Trägerstoffen, die zur Herstellung von Pudern oder wettable-powder geeignet sind.

Geeignete Trägerstoffe sind anorganische Trägerstoffe wie z. B. Talkum, Kaolin, Calciumcarbonat, Silikate, Bentonite, ferner organische Trägerstoffe wie z. B. Stärken, z. B. Reisstärke, Zucker, Zellulose und Zellulosederivate.

Die Mittel werden gegebenenfalls unter Zusatz von Netzmitteln durch Vermischen des oder der Wirkstoffe mit den Trägerstoffen hergestellt. Geeignete Netzmittel sind z.B. die weiter oben angeführten Emulgatoren.

Mittel, die verräuchert werden, enthalten den Wirkstoff in Konzentrationen von $10^{-7}$ - 2 Gew.-% pro 100 g Trägermaterial. Als Trägermaterial dient das übliche Material für Räucherpräparate.

Mittel, durch die der Wirkstoff verdampft wird, sind z.B. Trägermaterialien, die mit wirkstoffhaltigen Mitteln imprägniert sind oder in die der Wirkstoff eingearbeitet ist. Bevorzugt sind mit wirkstoffhaltigen Mitteln getränkte Papier-, Pappe-, Zellulose-, Stoff-, Filz-, Flies-, Lederplättchen oder -folien, die z.B. durch eine Wärmequelle erhitzt werden können. Als Wärmequelle seien die für Verdampferplättchen üblichen elektrischen bzw. batteriebetriebenen Verdampferöfchen genannt.

Besonders vorteilhaft ist es, Mittel einzusetzen, in die der Wirkstoff eingearbeitet oder auf die er aufgebracht ist und die ohne zusätzliche Wärmequelle wirken. Damit läßt sich die Behandlung besonders einfach durchführen. Die Mittel werden einfach in die Wohnung der Insekten eingeführt.

Die Behandlung wird durch Entfernung des Mittels beendet. Dies verhindert, daß die Parasiten ständig abnehmenden Wirkstoffkonzentrationen ausgesetzt sind. Dadurch wird einer Resistenzbildung bei den Parasiten vorgebeugt.

Die lang anhaltende Wirkstoffabgabe dieser Mittel erlaubt eine Langzeittherapie, die auch den Nachwuchs aus der Brut der Insekten erfaßt.

Der Wirkstoff kann bei diesen Mitteln in Trägerstoffen enthalten oder eingearbeitet sein oder in geeigneter Form auf Trägerstoffen aufgebracht sein.

Trägerstoffe sind Formkörper, die am oder in der Insektenwohnung angebracht werden. Auch Teile der Wohnung können aus Material geformt werden, in das der Wirkstoff eingearbeitet ist oder auf dessen Oberfläche der Wirkstoff aufgebracht ist oder das mit Wirkstoff getränkt oder imprägniert ist. Bevorzugt sind z.B. bei Bienen Schiede, die zwischen die Waben eingeschoben werden und die mit wirkstoffhaltigem Mitteln behandelt worden sind oder in die der Wirkstoff eingearbeitet ist.

Als Trägerstoffe können natürliche oder synthetische Trägerstoffe dienen. Natürliche Trägerstoffe sind z.B. Holz, Holzverarbeitungsprodukte, Pappe, Papier, Gummi, Kautschuk, Filz, Metall, Glas, Porzellan, keramische Materialien, Synthetische Trägerstoffe sind z.B. Kunststoffe auf Basis Polyvinyl, PVC, Polyacrylat, Polymethacrylat, Epoxid, Polyurethan, Polyester, Polyamid, Cellulose und deren Derivate, Polyethylen, Polypropylen, synthetischer Kautschuk.

Als Trägerstoffe kommen aber auch Schichten in Frage, die auf einen festen oder flexiblen Untergrund aufgebracht sind. Solche Schichten können saugfähig sein und mit wirkstoffhaltigen Mitteln behandelt werden. Sie können aber auch nicht saugfähig sein und den Wirkstoff eingearbeitet enthalten. In der Regel handelt es sich bei diesen Schichten um haftfähige Polymere, denen gegebenenfalls inerte Füllsubstanzen zugesetzt werden. Als Polymere werden dabei die Lackrohstoffe der Farbenindustrie sowie z. B. Cellulosederivate, Acrylate und Methacrylate eingesetzt.

Als Beispiele für Füllstoffe zur Herstellung saugfähiger Schichten seien genannt: Kaolin, Calciumcarbonat, Silikate, Bentonite, Cellulose, Cellulosederivate, Stärke, Holzpulver. Der Wirkstoff ist dabei entweder bereits in schichtbildendes Material eingearbeitet oder die Schicht wird nachträglich z. B. mit dem oben beschriebenen zu versprühenden Mittel getränkt oder imprägniert oder besprüht.

Schichten, die den Wirkstoff eingearbeitet enthalten, können auch durch wirkstoffhaltige Anstriche oder Lacke gebildet werden. Diese enthalten den Wirkstoff in einer Konzentration von 0,00001 - 10, bevorzugt 0,001 - 1 Gewichtsprozent neben der üblichen Beschichtungsgrundmasse. Bevorzugt werden

Dispersionsanstriche und -lacke als Beschichtungsgrundmasse eingesetzt.

Schichten, die den Wirkstoff eingearbeitet enthalten, können aber auch Folien, Streifen, Bänder sein, die ein- oder mehrschichtig, sowie gegebenenfalls selbstklebend ausgebildet sind.

So kann eine wirkstoffhaltige Selbstklebefolie z. B. aus einer Klebeschicht, einer flexiblen Trägerschicht, einer wirkstoffhaltigen flexiblen Trägerschicht, einer wirkstoffreien flexiblen Deckschicht bestehen. Die einzelnen Schichten bestehen aus an sich bekannten Polymermaterialien, die für die Herstellung solcher Schichten geeignet sind.

Wie bereits erwähnt, kann der Wirkstoff in diesen Formkörpern eingearbeitet enthalten sein. Diese enthalten den Wirkstoff in Konzentrationen von 0,00001 - 10 Gew.-%, bevorzugt 0,0001 - 1 Gew.-%, bezogen auf das Grundmaterial des Formkörpers.

Geeignete Formkörper sind Streifen, Bänder, Platten, aber auch, wie weiter oben erwähnt, Bauteile.

Für die Herstellung der erfindungsgemäßen Formkörper können Polyvinylharze, Polyacrylate, Epoxyharze, Cellulose, Cellulosederivate, Polyamide und Polyester verwendet werden, die mit den obengenannten Wirkstoffen ausreichend verträglich sind. Die Polymeren müssen eine ausreichende Festigkeit und Biegsamkeit haben, um beim Formen nicht zu reißen oder brüchig zu werden. Sie müssen eine ausreichende Wanderung der Wirkstoffe an die Oberfläche des Formkörpers zulassen.

Typische Vinylharze sind beispielsweise Polyvinylhalogenide, wie Polyvinylchlorid, Polyvinylchlorid-Vinylacetat und Polyvinylfluorid; Polyacrylat- und Polymethacrylatester, wie Polymethylacrylat und Polymethylmethacrylat; und Polyvinylbenzole, wie Polystyrol und Polyvinyltoluol.

Für die Herstellung der erfindungsgemäßen Formkörper auf der Basis Polyvinylharz sind die Weichmacher geeignet, die üblicherweise zum Weichmachen von festen Vinylharzen verwendet werden. Der verwendete Weichmacher hängt von dem Harz und seiner Verträglichkeit mit dem Weichmacher ab. Geeignete Weichmacher sind beispielsweise Ester von Phosphorsäure, wie Tricresylphosphat, Ester von Phthalsäure, wie Dimethylphthalat und Dioctylphthalat, und Ester von Adipinsäure, wie Diiosbutyladipat. Es können auch andere Ester, wie die Ester von Azelainsäure, Maleinsäure, Ricinolsäure, Myristinsäure, Palmitinsäure, Ölsäure, Sebacinsäure, Stearinsäure und Trimellithsäure, sowie komplexe lineare Polyester, polymere Weichmacher und epoxydierte Sojabohnehöle verwendet werden. Die Menge des Weichmachers beträgt etwa 10 bis 50 Gew.-%, vorzugsweis etwa 20 bis 45 Gew.-% der gesamten Zusammensetzung.

In den Formkörpern können noch weitere Bestandteile, wie Stabilisierungsmittel, Schmiermittel, Füllstoffe und Färbematerialien, enthalten sein, ohne daß dadurch die grundlegenden Eigenschaften der Zusammensetzung verändert werden. Geeignete Stabilisierungsmittel sind Antioxydationsmittel und Mittel, die den Formkörper vor ultravioletter Strahlung und unerwünschtem Abbau während der Bearbeitung, wie Strangpressen schützen. Einige Netzmittel wie epoxydierte Sojabohnenöle, dienen außerdem als sekundäre Weichmacher. Als Schmiermittel können beispielsweise Stearate, Stearinsäure und Polyethylen mit niedrigem Molekulargewicht verwendet werden. Diese Bestandteile können in einer Konzentration bis zu etwa 20 Gew.-% der gesamten Zusammensetzung verwendet werden.

Bei der Herstellung der erfindungsgemäßen Formkörper auf Vinylharzbasis werden die verschiedenen Bestandteile nach bekannten Mischverfahren trocken gemischt und nach bekannten Strangpreß- oder Spritzgußverfahren formgepreßt.

Die Wahl des Verarbeitungsverfahrens zur Herstellung der erfindungsgemäßen Formkörper richtet sich technisch grundsätzlich nach den rheologischen Eigenschaften des Formkörpermateriales und der Form des gewünschten Gebildes. Die Verarbeitungsverfahren können nach der Verarbeitungstechnologie oder nach der Art der Formgebung eingestellt werden. Bei der Verfahrenstechnologie kann man die Verfahren nach den bei ihnen durchlaufenen rheologischen Zuständen unterteilen. Danach kommen für viskose Formkörpermaterialien Gießen, Pressen, Spritzen und Auftragen und für elastoviskose Polymere Spritzgießen, Strangpressen (Extrudieren), Kalandrieren, Walzen und gegebenenfalls Kanten in Frage. Nach Art der Formgebung eingeteilt, lassen sich die erfindungsgemäßen Formkörper durch Gießen, Tauchen, Pressen, Spritzgießen, Extrudieren, Kalandrieren, Prägen, Biegen, Tiefziehen etc. herstellen.

Diese Verarbeitungsverfahren sind bekannt und bedürfen keiner näheren Erklärung. Im Prinzip gelten für Polymere wie Polyamide und Polyester die Erläuterungen, die oben beispielhaft für Polyvinylharze gemacht wurden.

Mittel die durch eine systemische Wirkung über die Hämolymphe der Insekten wirken, sind z.B. Futterstoffe die die Wirkstoffe enthalten. Als solche seien genannt: Zuckergranulate, zuckerhaltige Mischungen, Lösungen, Suspensionen oder Emulsionen. Diese enthalten Wirkstoffkonzentrationen von 0,5 - 20 Gew.-%, vorzugsweise von 1 - 10 Gew.-%. Diese Mischungen werden mit Wasser oder Zuckerlösung auf Anwendungskonzentrationen des Wirkstoffs von $10^{-8}$ - 1 Gew.-%, bevorzugt 0,0001 - 0,01 Gew.-%, besonders bevorzugt auf 0,0001 - 0,005 Gew.-% weiter verdünnt.

Ferner seien genannt anwendungsfertige Futterteige oder Knete die den Wirkstoff in der Anwendungskonzentration neben Zucker und Stärke enthalten.

Besonders bevorzugt sind Mittel für eine Anwendung der Wirkstoffe im Trinkwasser. Dafür kommen wassermischbare Lösungen der Wirkstoffe, die ein oder mehrere polare Lösungsmittel enthalten und alkalisch reagieren, in Frage.

Zur Herstellung solcher Lösungen wird der Wirkstoff in einem polaren, wasserlöslichen Lösungsmittel

gelöst, welches entweder alkalisch reagiert oder dem eine alkalische wasserlösliche Substanz zugefügt wird. Letztere wird zweckmäßigerweise ebenfalls im Lösungsmittel gelöst, kann aber auch in dem Lösungsmittel suspendiert sein und sich erst im Trinkwasser lösen. Dabei soll das Trinkwasser nach Zusatz der Wirkstofflösung einen pH-Wert von mehr als 7, vorzugsweise aber einen pH-Wert größer als pH 8 und kleiner als 11 haben.

Das Trinkwasser kann einen Zuckergehalt (Glukose) von 0,1 bis 5 Gew.-%, bevorzugt von ca. 1 Gew.-% haben.

Die Lösung des Wirkstoffkonzentrats sollte einen pH von 11 nicht überschreiten.

Die Konzentration des Wirkstoffs kann im Bereich von 0,5 bis 50 % liegen, vorzugsweise aber in einem Bereich von 1 bis 25 %.

Als Lösungsmittel kommen alle wasserlöslichen Lösungsmittel in Betracht, in denen der Wirkstoff in genügender Konzentration löslich ist und die physiologisch unbedenklich sind.

Dies sind aus der Reihe der Alkohole ein- und mehrwertige wie z.B. Ethylalkohol, Isopropylalkohol, Benzylalkohol, Glycerin, Propylenglykol, Polyethylenglykole, Poly(oxyethylen)-poly(oxypropylen)-Polymere, basische Alkohole wie z.B. Mono-, Di- und Triethanolamin.

Außerdem sind geeignet Ketone z.B. Aceton oder Methylethylketon und aus der Reihe der Ester z.B. Milchsäureethylester. Andere Lösungsmittel wie N-Methylpyrrolidon, Dimethylacetamid, Dimethylformamid können ebenfalls eingesetzt werden.

Als Basen zur Einstellung des alkalischen pH-Wertes sind vorzugsweise organische Basen einzusetzen, z.B. basische Aminosäuren wie L- bzw. D,L-Arginin, L- bzw. D,L-Lysin, Methylglucosamin, glucosamin, 2-Amino-2-hydroxymethylpropandiol-(1,3), Cholin, Piperazin. Auch Diamine sind hier geeignet z.B. bildet N,N,N',N'-Tetrakis-(2-hydroxypropyl)-ethylen-diamin oder Polyether-tetrol auf der Basis Ethylendiamin (M.G. 480-420, OH-Index 432-467) ebenfalls klare Lösungen im angegebenen pH-Bereich aus. Auch anorganische Basen können eingesetzt werden, z.B. Ammoniak oder Natriumcarbonat - gegebenenfalls unter Zugabe von Wasser.

Substanzen, die sonst als Emulgatoren oder Solubilisatoren verwendet werden und in Wasser kolloidal löslich sind, können in diesem Falle wie polare Lösungsmittel eingesetzt werden, sofern ihnen noch ein basischer Hilfsstoff zugemischt wird.

Zur Herstellung der Lösungen werden die Substanzen in einen Behälter mit Rührwerk eingewogen und dann unter Erwärmen solange gerührt, bis eine klare Lösung entstanden ist.

Wassermischbare Lösungen der Wirkstoffe zur Anwendung im Trinkwasser sind z.B.:

<u>Beispiel 1</u>

2,5 g Toltrazuril werden zu 100 ml in Triethanolamin unter Erwärmen gelöst.

Die klare Lösung hat nach Verdünnen mit Wasser im Verhältnis 1:10 einen pH-Wert von 10,2.

<u>Beispiel 2</u>

2,5 g Toltrazuril und 12,5 g Milchsäure werden zu 100 ml in Triethanolamin unter Erwärmen und Rühren gelöst.

Nach Verdünnen der Lösung mit Wasser im Verhältnis 1:10 beträgt der pH-Wert 8,3.

<u>Beispiel 3</u>

10,0 g Toltrazuril wird zu 100 ml Monoethanolamin gelöst.

Die Klare Lösung hat nach Verdünnen mit Wasser im Verhältnis 1:10 einen pH-Wert von 11.

<u>Beispiel 4</u> Toltrazuril5,0 g
Propylenglykol50,0 g
Natriumcarbonat5,0 g
Wasserad 100 ml
pH der Lösung 9,9.

<u>Beispiel 5</u> Toltrazuril2,5 g
Natriumcarbonat5,5 g
Polydiol 200ad 100 ml

Der Wirkstoff wird in Polidiol gelöst und das Natriumcarbonat darin suspendiert. PH-Wert nach Verdünnen mit Wasser im Verhältnis von 1:10 von 9.

Beispiele für Formulierungen zum Versprühen, die vor Anwendung mit Wasser auf die Anwendungskonzentration verdünnt werden:

Beispiel 6 Toltrasuril20 g.
Emulgator Toximul®
(Mischung aus Alkylbenzolsulfonat-Ca und nichtionogenen Emulgatoren und Methanol mit Hydrophil/Lipophil Balance HLB-Wert: 10)7 g
Emulgator Toximul S®
(Mischung aus Alkylbenzolsulfonat Ca und nichtionogenem Emulgator, Methanol mit Hydrophil/Lipophil Balance HLB-Wert: 10)5 g

Beispiel 8 Toltrazuril5 g
Emulgator Atlox®
(Gemisch aus Polyoxyethylenether, Polyoxyglycerid, Alkylarylsulfonat - sehr leicht wasserlöslich)4 g
Emulgator Atlox 3404®
(Gemisch aus Polyoxyethylenalkylarylether, Alkylarylsulfonat - gbldet Emulsion in Wasser)2 g

Beispiel 10 Wirkstoff:
Toltrazuril0,5 g
Netzmittel:
Emulvin W® (Alkylarylpolyglykolether)3,0 g
Wasserad 100 ml

Beispiel für ein Stäubemittel ist:

Beispiel 11

1 g Toltrazuril wird mit 99 g Talkum gründlich vermischt. Von dieser Mischung werden 5 g mit 95 g Talkum gründlich vermischt.

**Beispiel für einen PVC-Formkörper:**

**Beispiel 12**

| | |
|---|---|
| Toltrazuril | 0,5 g |
| Isobutyladipat | 15,5 g |
| Dialkylpythalat | 8,0 g |
| Polyoxyethyliertes Rizinusöl | 2,0 g |
| Stearinsäure | 0,8 g |
| Farbstoff | 0,1 g |
| Polyvinylchlorid | 73,1 g |
| | 100,0 g |

100,0 kg dieser Mischung werden in einem Mischer - in der üblichen Arbeitsweise für Weich-PVC - homogen gemischt.

Diese Mischung wird auf einer Spritzgußmaschine zu einem Wabenschied verarbeitet. Gewicht des Schieds: 86,0 g.

Obige Mischung wird statt mit 0,5 mit 0,25 g Wirkstoff hergestellt und auf einer entsprechenden Kalandrier-Vorrichtung zu einer Folie von der Größe eines DIN-A4-Blattes ausgewalzt. Gewicht der Folie 50,0 g. Das Blatt wird z.B. in den Bienenstock gelegt.

Beispiel für einen beschichteten Träger

Beispiel 13

Auf einer Folie aus 2 mm dickem Polyethylen wird eine Lösung von Toltrazuril im Emulgator Span 20® Atlas und Ethanol mittels einer Rakel gleichmäßig aufgetragen. Die Lösung wird so eingestellt, daß 1 mg Toltrazuril pro 100 $cm^2$ Oberfläche und 0,5 mg Emulgator pro 100 $cm^2$ aufgetragen werden. Das Lösungsmittel wird abgedampft und die Folie in beliebiger Form ausgestanzt.

Beispiele für einen getränkten Träger mit Polymer(=Lack)-Zusatz

Beispiel 14

Mit Kieselgur beschichtete Aluminiumfolien werden mit einer Lösung aus Toltrazuril und Polyvinylalkohol so behandelt, daß nach dem Trocknen auf der Folie pro 100 $cm^2$ 5 mg Toltrazuril und 20 mg Polyvinylalkohol zurückbleiben.

Die Trägerformen der letzten beiden Beispiele können mit einem Haftkleber versehen sein. Nach Abziehen des Klebschutzes lassen sie sich im Insektenwohnungen einfach einkleben.

Beispiele für ein Granulat zur Fütterung

Beispiel 15

0,5 kg Toltrazuril werden in 7,5 l Ethanol unter vorsichtigem Erwärmen gelöst und in einem Mischgranulator auf 99,5 kg Zucker gegossen während der Mischer läuft. Der vom Alkohol feuchte, gleichmäßig getränkte Zucker wird getrocknet und gegebenenfalls gesiebt. 1,0 g des Granulates wird vor Gebrauch in 100 ml Wasser zu einer Zuckerlösung aufgelöst, die den Insekten zur Nahrung dient.

Beispiel A:

10 adulte Schlupfwespen (Pimpla turionella) die mit Nosema apis verseucht waren, wurden 48 Stunden lang bei 22°C gehalten und über das Trinkwasser ad lib. mit einer 1 % Zuckerlösung, die 20 ppm Toltrazuril enthielt, behandelt.

Am Ende des Versuchs lebten 9 Tiere die abgetötet wurden. Mikroskopische Untersuchung ergab Schädigung (z.T. Cytolyse) der in ihnen parasitierenden Protozoen.

Bei einer Kontrollgruppe die nur mit Zuckerwasser (1 %) behandelt wurde, starben 7 von 10 Tieren. Die überlebenden Tiere waren stark von Nosema apis befallen.

Beispiel B:

Abdomenteile von Pimpla turionella, die vorher mikroskopisch auf das Vorhandensein von Nosema apis untersucht worden waren, wurden in 30 ml physiologische Kochsalzlösung gelegt, die 20 ppm Toltrazuril enthielt. Nach 12 Stunden Inkubation bei 20°C wurden die Teile erneut lichtmikroskopisch untersucht. Es zeigten sich schwere Schädigungen der Protozoen. In einer Kontrolle ohne Wirkstoff konnten keine Schädigungen der Protozoen festgestellt werden.

**Patentanspruch**

1.Verwendung von Triazintrionen der Formel (I)

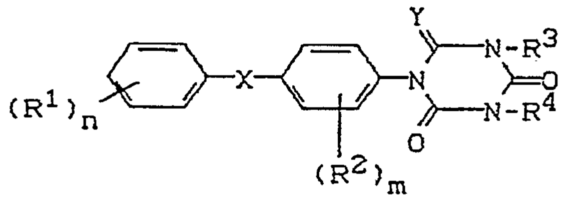

(I)

in welcher X für O oder S steht, Y für O oder S steht, $R^1$ für gleiche oder verschiedene Reste aus der Gruppe Halogen, Nitro, CN, Amino, Alkyl, Halogenalkyl, Alkoxy, Halogenalkoxy, Alkylthio, Halogenalkylthio, Alkylsulfonyl, Alkylsulfinyl, Halogenalkylsulfonyl, Halogenalkylsulfinyl, Acyl, Carboxy, Carbonylamino, Carbonylalkoxy, Carbamoyl, Sulfamoyl steht, $R^2$ für gleiche oder verschiedene Reste aus der bei R1 angegebenen Gruppe von Substituenten, n und m für ganze Zahlen von 0 bis 3 stehen, $R^3$ für Wasserstoff oder Alkyl steht, $R^4$ für Wasserstoff oder Alkyl steht, zur Herstellung von Mitteln zur Bekämpfung von Protozoen bei Insekten.

**Claim**

1. Use of triazinetriones of the formula (I)

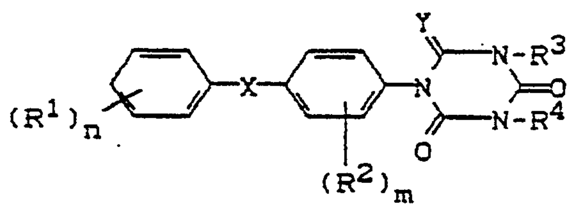

(I)

in which X represents O or S, Y represents O or S, the symbols $R^1$ represent identical or different radicals from the group comprising halogen, nitro, CN, amino, alkyl, halogenoalkyl, alkoxy, halogenoalkoxy, alkylthio, halogenoalkylthio, alkylsulphonyl, alkylsulphinyl, halogenoalkylsulphonyl, halogenoalkylsulphinyl, acyl, carboxy, carbonylamino, carbonylalkoxy, carbamoyl and sulphamoyl, the symbols $R^2$ represent identical or different radicals from the group of substituents given for $R^1$, n and m represent integers from 0 to 3, $R^3$ represents hydrogen or alkyl and $R^4$ represents hydrogen or alkyl, for thepreparation of agents for combating protozoa in insects.

**Revendication**

1. Utilisation de triazinetriones de formule (I)

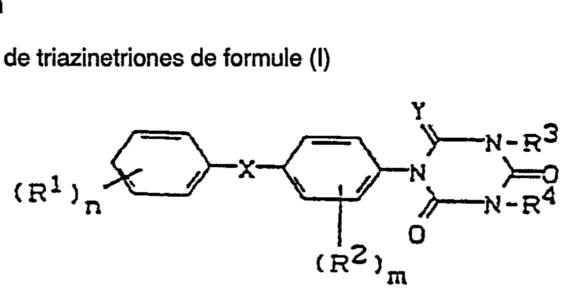

(I)

dans laquelle: X représente O ou S, Y représente O ou S, $R^1$ représente des radicaux identiques ou différents choisis parmi le groupe comprenant: un atome d'halogène, un groupe nitro, un groupe CN, un groupe amino, un groupe alkyle, un groupe halogénalkyle, un groupe alcoxy, un groupe halogénalcox, un groupe alkylthio, un groupe halogénalkylthio, un groupe

alkylsulfonyle, un groupe alkylsulfinyle, un groupe halogénalkylsulfonyle, un groupe halogénalkylsulfinyl, un groupe acyle, un groupe carboxyle, un groupe carbonylamino, un groupe carbonylalcoxy, un groupe carbamoyle, un groupe sulfamoyle,

$R^2$ représente des radicaux identiques ou différents, choisis parmi le groupe de substituants indiqué pour $R^1$,

n et m représentent des entiers de 0 à 3,

$R^3$ représente un atome d'hydrogène ou un groupe alkyle,

$R^4$ représente un atome d'hydrogène ou un groupe alkyle,

pour la préparation d'agents pour la lutte contre les protozoaires chez les insectes.